# EUROPEAN PATENT SPECIFICATION

(11) **EP 4 126 137 B1**
(45) Date of publication and mention of the grant of the patent: **04.12.2024**
(21) Application number: 21717529.8
(22) Date of filing: 19.03.2021
(51) Int. Cl.: A61M 11/06, A61M 15/00, A61M 15/02

(54) **INHALATION DEVICE**
INHALATIONSVORRICHTUNG
DISPOSITIF D'INHALATION

(30) Priority: 23.03.2020 BE 202005187
(43) Date of publication of application: 08.02.2023
(73) Proprietor: Coigniez-Walraevens, 9406 Outer (BE)
(72) Inventor: COIGNIEZ, Guillaume, 9406 Outer (BE); COIGNIEZ, Peter, 9406 Outer (BE)
(74) Representative: Chielens, Kristof
(86) International application number: PCT/IB2021/052306
(87) International publication number: WO 2021/191756

(56) References cited:
- WO-A1-2020/002351
- RU-C1- 2 111 025
- US-A- 5 603 314
- US-B2- 8 387 895

## Description

This invention relates on the one hand to a device for producing an aerosol for inhalation by a person, comprising a fluid reservoir for a fluid, a nebulizing element provided in order to convert the fluid from the fluid reservoir into an aerosol, and a dispensing tube with a discharge opening along which the aerosol produced is dispensable so that it can be inhaled by a person. This device relates in particular to a device for the treatment of acute and chronic lung disorders.

On the other hand, this invention relates to a method for obtaining an aerosol suitable for the treatment of acute and chronic lung disorders.

At present, there are already various methods available for treating acute and chronic lung disorders, on the one hand, by means of drugs (antibiotics, corticosteroids, antihistamines, antitussives, etc.) administered orally, by inhalation, or parenterally. On the other hand, there are also mechanical methods, such as e.g. breath-holding therapy, tapotage, and autogenous drainage.

Aerosol therapy, in which a drug-containing fluid is administered in aerosol form to both children and adults with asthma or respiratory tract infections, is generally known. In this therapy, one uses a so-called nebulizer, which converts a drug solution into a fog or mist. The mist formed can then be inhaled via a mouthpiece (or an oronasal mask), which allows the nebulized drug to easily reach the respiratory tract. Commonly used drugs that can be nebulized include anti-inflammatories, mucolytics, bronchodilators and antibiotics.

US patent publication US 5 603 314 A describes a device for producing an aerosol for inhalation by a person comprising a fluid reservoir for a fluid, a nebulizing element provided in order to convert the fluid from the fluid reservoir into an aerosol, and a dispensing tube with a discharge opening along which the aerosol produced is dispensable so that it can be inhaled by a person.

RU 2 111 025 discloses an apparatus for treating respiratory and lung diseases comprising a laser emitter to irradiate the mucosa of the respiratory tract, wherein the irradiation is carried out by inhaling an aerosol irradiated with a laser beam.

The present application is based on the principle of aerosol therapy, wherein the applicant surprisingly found that a nebulizer can be converted into a device for producing an aerosol, which aerosol has a positive effect on the health of the user and is preferably used for the treatment of acute and chronic lung disorders.

This object of the invention is therefore to provide a device for producing an aerosol for inhalation by a person that can be used in particular by people with acute and/or chronic lung disorders.

The object of the invention is achieved by providing a device for producing an aerosol for inhalation by a person, according to claim 1. By irradiating the aerosol produced by the nebulizing element, preferably a mixture of fluid particles in a gas, with laser light, the aerosol absorbs part of the energy from the laser light. When the aerosol treated in this manner is then inhaled, it comes into contact with the mucous membrane, where it promotes tissue repair, among other actions.

In a preferred embodiment of the device in accordance with the invention, the laser is provided in order to generate a light beam with a wavelength of between 500 nm and 1000 nm. Preferably, the laser is provided in order to generate a light beam with a wavelength of between 550 nm and 850 nm. More particularly, the laser is provided in order to generate a light beam with a wavelength of 650 nm or 808 nm. A wavelength of 650 nm belongs to the visible light spectrum and is advantageous in that there is less risk of eye injury, e.g. if someone were to look through the dispensing tube in the direction of the laser. Moreover, it is advantageous in that at the same intensity, irradiation of the aerosol with a shorter wavelength generates a higher energetic response with respect to the tissue with which the aerosol comes into contact. The laser is preferably a diode laser. The diode laser preferably comprises two laser diodes, each diode having a capacity of 5 mW.

In an alternative embodiment of the device in accordance with the invention, the laser is provided in order to generate a light beam with a wavelength of between 400 nm and 500 nm. Preferably, the laser is provided in order to generate a light beam with a wavelength of 470 nm. A device provided with such a laser in order to generate a light beam with a wavelength of between 400 nm and 500 nm is particularly suitable for the treatment of both bacterial and fungal infections.

Preferably, the dispensing tube should converge in the direction of the discharge opening. The dispensing tube comprises a tapered part, wherein the discharge opening is provided in the tapered part. The tapered configuration of the end makes it possible to create a turbulent flow of the aerosol inside the tube, at the level of the transition to the tapered part of the tube, which allows the aerosol mist to be held in place for a longer period and thus to be irradiated for a longer period.

In a particular embodiment of the device in accordance with the invention, the dispensing tube comprises a supply channel for aerosol and an uptake channel suitable for holding the laser. The supply and uptake channels are preferably located in the first part, at the beginning of the dispensing tube. The said first part may constitute an integral part of the dispensing tube or may be configured as a separate component (attachment) that can be placed on a second tube-shaped part. The two parts together form the dispensing tube. The supply and uptake channels are located at virtually the same level in the dispensing tube, wherein the supply channel is provided above (or below) the uptake channel. In practice, the supply channel should be located above, as this allows the aerosol to be better distributed in the dispensing tube. At the level of the supply and uptake channels, the dispensing tube should have an inner diameter of 40 to 42 mm, and at the level of the discharge opening, the diameter should be 34 to 36 mm.

The nebulizing element operates on the basis of compressed air, and for this purpose, the device in accordance with a particular embodiment comprises a compressed air source, a compressed air line for transporting compressed air from the compressed air source to the fluid reservoir, and a suction line for aspirating ambient air.

In a most preferable embodiment, the device in accordance with the invention comprises a housing containing a filter chamber for filtering the aspirated ambient air. The filter chamber is preferably suitable for accommodating an (exchangeable) air filter.

A further subject matter of this invention relates to a method for obtaining an aerosol suitable for the treatment of acute and chronic lung disorders, wherein the method comprises the following steps:
- conversion of a fluid into an aerosol; and
- irradiation of the aerosol formed with laser light having a wavelength of between 500 nm and 1000 nm according to claim 9.

An aerosol processed in this manner, preferably a mixture of fluid particles in a gas, is particularly suitable for the treatment of acute and chronic lung diseases. The above-mentioned fluid is preferably physiological saline, because such a fluid is neutral for the human body.

In accordance with a preferred method, the aerosol formed is irradiated with laser light having a wavelength of between 550 nm and 850 nm.

In an alternative method, the aerosol formed is irradiated with a laser light having a wavelength of between 400 nm and 500 nm. An aerosol treated in this manner is particularly suitable for the treatment of both bacterial and fungal infections.

The method in accordance with the invention is particularly suitable for application by means of the device in accordance with this invention.

The present invention will now be described in further detail by means of the following detailed description of a preferred embodiment of a device and method in accordance with this invention. This description is intended solely to give explanatory examples and to point out further advantages and special features thereof, and can thus by no means be interpreted as limiting the scope of application of the invention or the patent rights claimed in the claims.

In this detailed description, the attached drawings are referred to by means of reference numbers, wherein:
- ***Fig. 1*** *shows a perspective view of the device in accordance with the invention;*

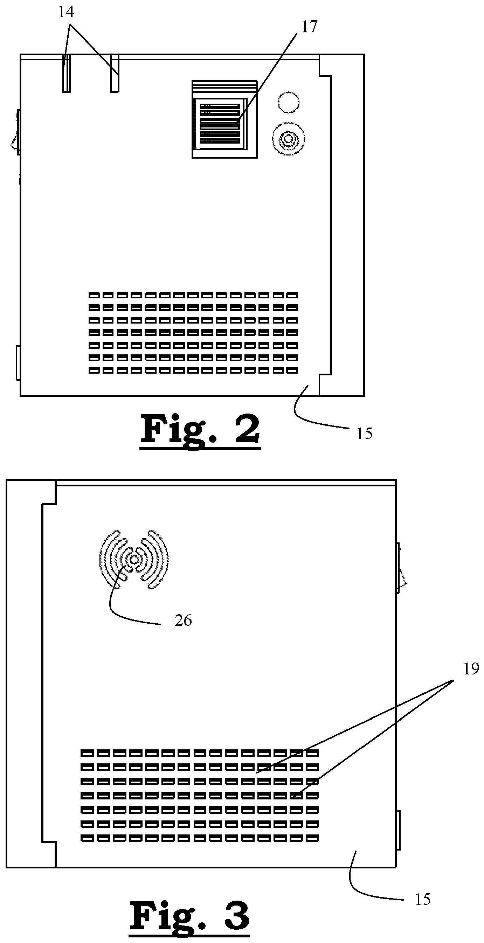

- ***Fig. 2****: is a left-side view of the device shown in* *Fig. 1**;*
- ***Fig. 3*** *is a right-side view of the device shown in* *Fig. 1**;*
- ***Fig. 4*** *is a rear view of the device shown in* *Fig. 1**;*
- ***Fig. 5*** *is an inside view of the device shown in* *Fig. 1**;*
- ***Fig. 6*** *shows the first part of the dispensing tube, wherein the supply channel for aerosol and the uptake channel suitable for holding the laser are provided;*
- ***Fig. 7*** *shows a top view of the part shown in* *Fig. 6**;*
- ***Fig. 8*** *is an illustration of a laser suitable for generating a light beam with which an aerosol is irradiated in the dispensing tube;*
- ***Fig. 9*** *is an illustration of the second part of the dispensing tube;*
- ***Fig. 10*** *shows a sectional view of the fluid reservoir with the nebulizing element.*

This invention relates to a device (1) for producing an aerosol for inhalation by a person, wherein so-called aerosol laser therapy is used. In this therapy, one is to inhale nebulized physiological saline irradiated with laser light, preferably red laser light. We know from quantum physics that laser light from the red and infrared regions (and thus also the energy associated therewith) is absorbed by water vapour. Water vapour is used as a vehicle for inhaling laser energy, not as an inhalation medication. The inhaled 'enriched' water vapor condenses on the mucosa of the respiratory tract, and the absorbed laser energy thus also comes into contact with the mucous membrane.

From this point on, the effect is analogous to the effect after direct irradiation with an infrared laser: at the molecular level, atoms and molecules are brought into an excited state by energetic stimulation. When they return to a stable state, the stored energy is released again, bringing an adjacent atom or molecule into an excited state. This gives rise to a chain reaction wherein the energy released can be used in each case to reduce inflammation and repair tissue. In addition to the supply of directly usable energy, capillary dilation is produced that promotes both the immune reaction and tissue repair.

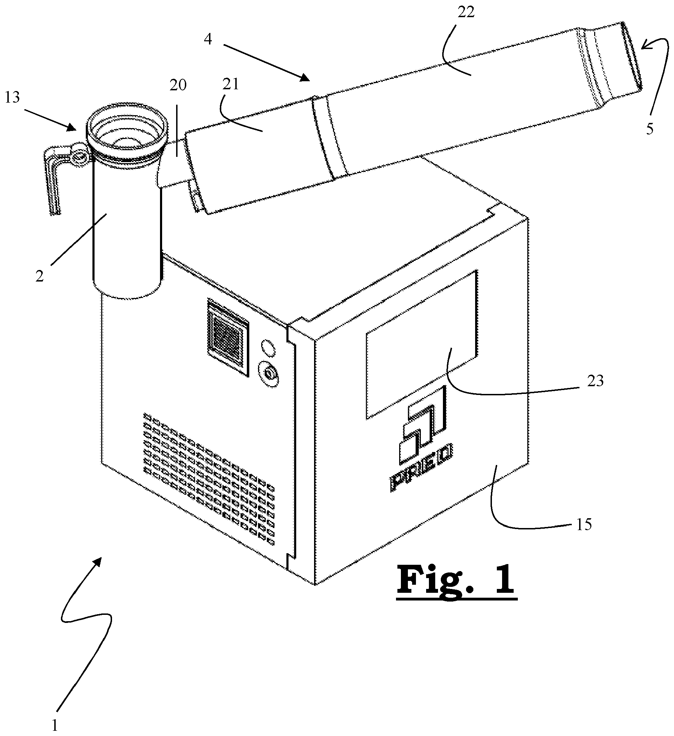

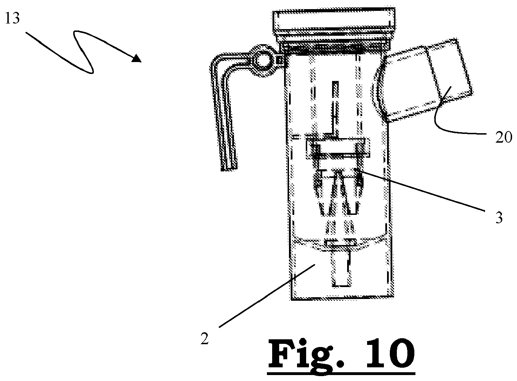

The device (1) in accordance with the invention is shown in Fig. 1 and comprises a fluid reservoir (2) suitable for the temporary storage of a fluid. The fluid used is preferably physiological saline. As shown in Fig. 10, a nebulizing element (3) is provided in the fluid reservoir (2) in order to convert the fluid from the fluid reservoir in a known manner into an aerosol. The fluid reservoir (2), together with a nebulizing element (3) that operates based on air flow, forms a nebulizing unit (13). The said nebulizing unit (13) is placed on a support (14) provided for this purpose in the housing (15) of the device (1). The support (14) is formed by two recesses in which a part of the nebulizing unit (13) can be placed.

The device (1) is further equipped with a dispensing tube (4) that is connected to the nebulizing unit (13). The dispensing tube (4) has a discharge opening (5) along which the aerosol produced leaves the device (1) so that it can be inhaled by a person. In the dispensing tube (4), the aerosol is irradiated with laser light from a laser (6). The laser is a diode laser and comprises in a preferred embodiment two laser diodes (7), each having a capacity of 5 mW, and in practice, a laser beam is generated with a wavelength of 650 nm. Of course, the wavelength of the laser beam may vary. Preference is given to a wavelength of between 500 nm and 1000 nm.

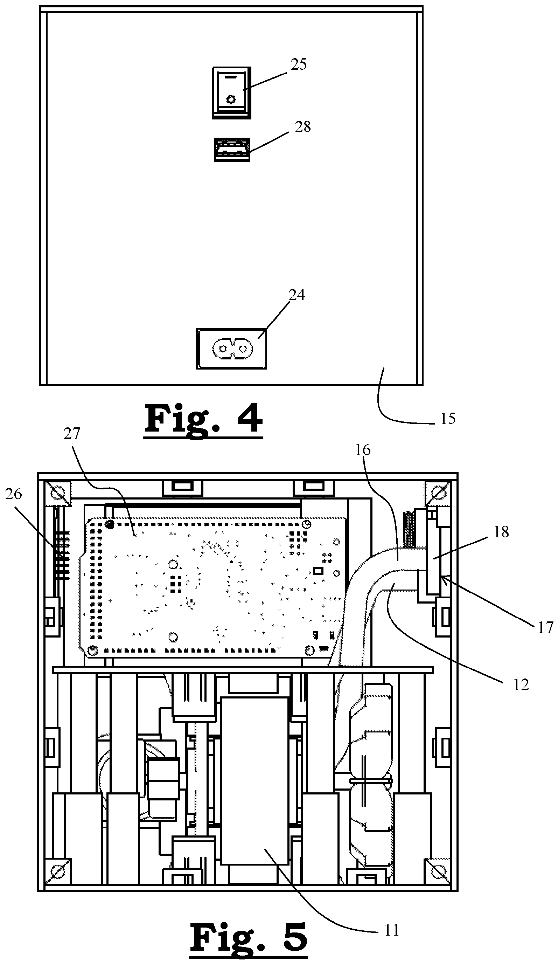

The fluid reservoir (2) is produced from high-quality PVC and has a capacity of 10 (± 0.5) ml. The nebulizing element (3) nebulizes the fluid provided in the fluid reservoir (2) in the known manner by means of compressed air. The nebulized fluid (aerosol) leaves the fluid reservoir via an outlet (20). In order to be able to nebulize the fluid, as shown in Fig. 5, the device (1) comprises a compressed air source (11) and a compressed air line (12) provided in the housing (15) in order to transport compressed air from the compressed air source (11) to the fluid reservoir (2). The compressed air source, for example, may be a standard oil-free piston compressor (pressure 150 KPa, flow rate ≥ 10 L/min). Of course, a suction line (16) for ambient air is also present in the housing (15). The ambient air comes into the housing (15) via a filter chamber (17), in which an (exchangeable) air filter (18) is placed in order to prevent particulate matter or other impurities from getting into the compressor. Various ventilation openings (19) are also present in the housing (15).

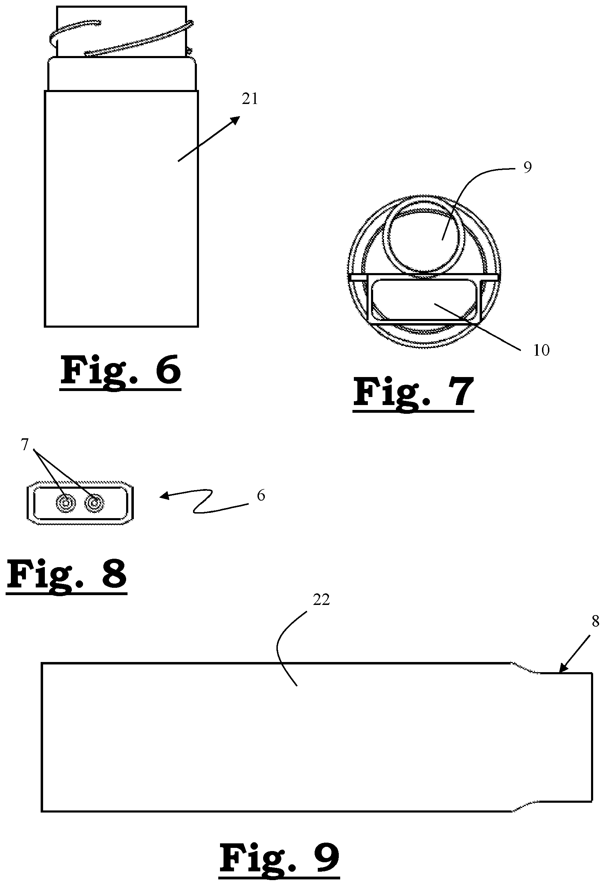

The dispensing tube (4) is placed on the outlet (20) of the fluid reservoir (2) so that the aerosol formed flows directly into the dispensing tube (4). The dispensing tube has a length of between 100 mm and 200 mm and should converge slightly in the direction of the discharge opening. In practice, the dispensing tube (4) has a diameter of approximately 45 mm at its beginning and a diameter of approximately 38 mm at its end (at the level of the discharge opening). The dispensing tube (4) may be configured as a single piece or may be composed of multiple parts (21; 22). In the device shown, the dispensing tube (4) is composed of two parts, particularly a first part (21) along which the aerosol formed comes in and a second part (22) wherein the discharge opening (5) is provided. In order to connect the first part (21) to the second part (22), the end of the first part (21), as shown in Fig. 6, may be provided with a screw thread.

The aerosol comes into the dispensing tube (4) via the first part (21). For this purpose, the first part (21) comprises a supply channel (9) (cf. Fig. 7). The first part (21) further comprises an uptake channel (10) wherein the laser (module) can be placed. This uptake channel (10) is preferably located below (but can also be above) the supply channel (9). The first part (21) is shorter than the second part. The first part (21) has a standard length of 7 to 8 cm.

The second part (22) of the dispensing tube (4) has a longer configuration, with the length of the second part (22) being approximately 14 cm to 17 cm. It is primarily in the second part (22) of the dispensing tube (4) that the aerosol is irradiated with laser light. The second part (22) also comprises a tapered part (8). In the tapered part (8), the discharge opening (5) of the dispensing tube (4) is provided. By means of a tapered configuration of the end, e.g. the last 3 cm, of the second part (22), it becomes possible to create a turbulent flow of the aerosol inside the tube, particularly at the level of the transition to the tapered part (8), which allows the aerosol mist to be held in place for a longer period and thus to be irradiated for a longer period. After the aerosol irradiated with laser light leaves the dispensing tube (4), it can be inhaled by a user.

Moreover, the device in accordance with the invention, particularly the housing (15), further comprises a control unit (27) for controlling the compressed air source (11) (compressor), a touch control panel (23), a power supply for the laser module, a plug connector (24), a USB port (28) and an on/off switch (25). The device (1) may also be equipped with an RFID module (26) that can be used to prevent unauthorised parties from using the device. Instead of an RFID module (26), one may also use a key lock.

The device (1) in accordance with the invention offers a broad range of possibilities for application, particularly in the treatment of:
- 1.: Bronchial asthma;
- 2.: Post-infectious cough;
- 3.: Small airways disease;
- 4.: Allergic asthma;
- 5.: COPD due to smoking;
- 6.: Bronchiectasis;
- 7.: Emphysema;
- 8.: Viral tracheobronchitis;
- 9.: Bacterial bronchitis;
- 10.: Eradication of multiresistant bacteria from sputum;
- 11.: Pulmonary sarcoidosis;
- 12.: Mucoviscidosis; and
- 13.: Repair of damaged lung tissue following pneumonia (e.g. COVID-19)

This list is not exhaustive. It is illustrative of the diversity of the spectrum of lung disorders in which the technology and device are applicable. Theoretically, one may expect a positive effect in any lung disease accompanied by inflammation and/or sputum production.

### 1. Bronchial asthma

The effect on bronchial asthma can be understood as that of detaching sputum from the airway walls, with the inflammation then being gradually suppressed until the amount of sputum produced is finally reduced to a normal level. Whether or not in combination with a long-acting β2 agonist, while discontinuing inhaled corticosteroids, this initially leads to a reduction in shortness of breath and coughing fits. The reduction in inflammation leads to less sputum production, which in turn results in less reflux and therefore also fewer superinfections. Spirometry shows an increase in FVC, FEV1 and Tiffeneau values.

In a patient dependent on inhaled corticosteroids in whom administration of inhaled corticosteroids is medically contraindicated, for example, following discontinuation of inhaled corticosteroids, the patient is free of attacks with 60 treatments per year, and has continued to be so for 26 months at present.

### 2. Post-infectious cough

Similarly to the effect on bronchial asthma, sputum is detached from the airway walls, while the inflammation is gradually suppressed until the amount of sputum produced is finally reduced to a normal level.

### 3. Small airways disease

Similarly to the effect on bronchial asthma, sputum is detached from the airway walls, while the inflammation is gradually suppressed until the amount of sputum produced is finally reduced to a normal level.

### 4. Allergic asthma

Aerosol laser therapy has also been shown to be useful in treatment of allergic asthma. On spirometry, this therapy shows better results than a combination of inhaled corticosteroids and a β2 agonist.

Because of the anti-inflammatory effect, the inflammatory reaction inherent in asthma is minimized, and wheezing, coughing and dyspnoea are suppressed.

### 5. COPD due to smoking

COPD patients suffer from hyperproduction of sputum. Aerosol laser therapy has a 'washing' effect on the sputum present, after which sputum production decreases. Spirometry shows an increase in FVC, FEV1 and Tiffeneau values.

This also limits the number of exacerbations.

### 6. Bronchiectasis

Patients with bronchiectasis (known to be irreversible), in which parts of the lungs are constantly dilated and irritated, are susceptible to recurrent infections. They benefit from this treatment primarily due to a bronchial lavage effect. The reduced amount of residual sputum then leads to fewer exacerbations and thus less need for antibiotics. Not only the inflammation-reducing effect is important; in addition, the effect of repairing and healing tissue also appears to lead to noteworthy results.

For example, in a patient known to have already suffered from bronchiectasis for more than 12 years, after 30 30-minute sessions of aerosol laser therapy, the radiologist reported that on high-resolution thoracic CT, bronchiectasis was no longer present, compared to high-resolution CT results from 2.5 years earlier, while the professor of pulmonology described it as limited.

In a patient who had shown clear bronchiectasis on a high-resolution CT scan conducted almost 12 years earlier, after 30 30-minute sessions of aerosol laser therapy, bronchiectasis was described in a report on recently-conducted HRCT as minimal.

It appears that in this case, one cannot rule out that the tissue-repairing effect of the laser energy applied to the mucosa may have played a role.

### 7. Pulmonary emphysema

Long-term treatment due to pronounced persistent densification in a pulmonary lobe following pneumonia caused supposedly irreversible emphysematous bullae to disappear. After 80 1-hour treatments, the emphysema was reported on HRCT to be only minimal.

### 8. Viral tracheobronchitis

A test was conducted in order to determine whether aerosol laser therapy should be continued or interrupted if a viral respiratory tract infection occurs during treatment. A case of viral bronchitis was completely supressed after 2 days (without any type of medication).

### 9. Bacterial bronchitis.

A test was also conducted in order to determine whether aerosol laser therapy should be continued or interrupted if a bacterial respiratory tract infection occurs during treatment. A case of bacterial bronchitis was completely cured after 8 days (without any type of medication).

### 10. Eradication of multiresistant/nosocomial bacterial from sputum

Patients experience great benefit from this treatment due to the effect of bronchial lavage. This allows the patient to gradually cough up infected material that is a breeding ground for bacteria, after which the inflammation is further suppressed. In combination with a long-acting β2 agonist, without any inhaled corticosteroids or antibiotics, this leads to a reduction in shortness of breath. Coughing fits decrease in intensity, duration and frequency. Lumps of sputum become ever smaller and less viscous until the production of sputum is finally reduced to a normal level. O₂ saturation again increases. The tissue-repairing effect of the laser energy deposited on the mucosa allows the inflammatory processes to be reduced to a minimum.

### 11. Pulmonary sarcoidosis

A patient with pulmonary sarcoidosis, who despite the use of a β2 agonist and an inhaled corticosteroid continued to complain of dyspnoea and showed O₂ saturation of 90-93%, progressed to a status in which the dyspnoea was barely present and O₂ saturation was about 97-98% without administration of any β2 agonists or inhaled corticosteroids.

### 12. Mucoviscidosis

Although the efficacy of this treatment in mucoviscidosis patients has not yet been proven, it can safely be expected from prior experience that the effects will be the same in mucoviscidosis patients. Congenital production of viscous sputum will of course remain unchanged, but it is possible that the sputum produced will be less viscous. However, the question of whether viscous sputum is the result of mucoviscidosis or is due to another cause cannot affect the patient's capacity to cough up and evacuate this sputum. There is no reason to assume that capillary dilation will not occur after aerosol laser treatment, and this will therefore not only have a healing effect on the mucosa, but will also possibly make the mucus more fluid and thus less viscous.

### 13. Repair of damaged lung tissue following pneumonia (e.g. COVID-19)

In this specific case, efficacy in the acute phase of COVID-19 pneumonia has now also been established. The effect can without doubt be described as quite clear and lead after only the first 30-minute treatment to a noticeable difference, which was experienced both by the patient (easier breathing, less feeling of constriction, significant improvement in coughing, spontaneous reports of good night-time sleep) (subjectively) and by the treating physician on auscultation (objectively).

It is also known that severe residual scarring after pneumonia, which was predicted by the pneumonologist to be permanent, was completely resorbed following intensive treatment, resulting in normalisation of the lung parenchyma on HRCT. In this specific case, spirometry showed a volume increase in forced vital capacity of 33 times the level considered the current pharmaceutical gold standard. There is no reason why the results in the case of lung damage following COVID-19 pneumonia should be any different.

### Conclusion:

Despite the already broad range of lung disorders in which the aerosol laser technique is applicable, one may expect, as mentioned above, a positive effect in any lung disease accompanied by inflammation and/or sputum production.

As it is known that aerosol laser therapy without the use of antibiotics is capable of healing bacterial infections and that the number of exacerbations is drastically reduced in chronic use, it is highly likely that the number of hospitalisations due to exacerbations will also be decreased.

Not only in the case of the chronic pulmonary patient (greater well-being, fewer exacerbations, less medication, fewer complications), but also from a health economic standpoint (fewer antibiotics, fewer hospitalisation days, the possibility of combatting chronic infections with multiresistant nosocomial microbes, less intensive care, reduction in the number or postponement of lung transplants, etc.), aerosol laser therapy, which is also inexpensive, appears to be capable of having a significant impact on the health care budget.

Treatment by ALT (red light, 650 nm) has not been capable of preventing *Candida* species (not C. *albicans*) from being grown from sputum cultures. Scientific studies have established that irradiation of the skin with blue light (400-500 nm) results in both antimicrobial and fungicidal effects.

For example, irradiation with blue light has a fungicidal effect on *Candida* and results in an antimicrobial effect on species such as *Pseudomonas aeruginosa,* which is a significant finding in view of the increasing resistance to current antibiotics.

In an initial qualitative test conducted in a dark room of a laser physics laboratory, the laser light appears to partially "travel along" with the aerosol. The following test is to be conducted with a sensitive performance meter.

If the blue light also travels along with the aerosol, this may be of significant further importance in the treatment of both bacterial and fungal infections.

The device is therefore equipped with a supplementary laser module, preferably a 470nm laser module.

## Claims

1. Device (1) for producing an aerosol for inhalation by a person, comprising a fluid reservoir (2) for a fluid, a nebulizing element (3) provided in order to convert the fluid from the fluid reservoir (2) into an aerosol, and a dispensing tube (4) with a discharge opening (5) along which the aerosol produced is dispensable so that it can be inhaled by a person, wherein the device (1) further comprises a laser (6) provided in order to generate a light beam suitable for irradiating the aerosol during its trajectory through the dispensing tube (4), wherein the dispensing tube (4) has a length of between 100 mm and 200 mm. and said dispensing tube (4) comprises a tapered part (8), wherein the discharge opening (5) is provided in the tapered part, in order to create a turbulent flow of the aerosol inside the tube at the level of the transition to the tapered part of the tube, allowing the aerosol to be held in place for a longer period and thus to be irradiated for a longer period.

2. Device (1) according to Claim 1, wherein the laser (6) is provided in order to generate a light beam with a wavelength of between 500 nm and 1000 nm.

3. Device (1) according to Claim 1 or 2, wherein the laser (6) is provided in order to generate a light beam with a wavelength of between 550 nm and 850 nm.

4. Device (1) according to Claim 1, wherein the laser (6) is provided in order to generate a light beam with a wavelength of between 400 nm and 500 nm.

5. Device (1) according to one of the preceding claims, wherein the laser (6) is a diode laser.

6. Device (1) according to one of the preceding claims, wherein the dispensing tube (4) comprises a supply channel (9) for aerosol and an uptake channel (10) suitable for holding the laser (6).

7. Device (1) according to one of the preceding claims, wherein the device (1) comprises a compressed air source (11), a compressed air line (12) for transporting compressed air from the compressed air source (10) to the fluid reservoir (2), and a suction line (16) for aspirating ambient air.

8. Device (1) according to Claim 7, wherein the device (1) comprises a housing (15) containing a filter chamber (17) for filtering the aspirated ambient air.

9. Method for obtaining an aerosol suitable for the treatment of acute and chronic lung disorders, which method comprises the following steps:
- conversion of a fluid into an aerosol; and
- irradiation of the aerosol formed with laser light having a wavelength of between 500 nm and 1000 nm wherein the formed aerosol is irradiated during its trajectory through a dispensing tube (4) with a discharge opening (5) along which the aerosol produced is dispensable so that it can be inhaled by a person, wherein the dispensing tube (4) has a length of between 100 mm and 200 mm. and said dispensing tube (4) comprises a tapered part (8), wherein the discharge opening (5) is provided in the tapered part, in order to create a turbulent flow of the aerosol inside the tube at the level of the transition to the tapered part of the tube, allowing the aerosol to be held in place for a longer period and thus to be irradiated for a longer period.

10. Method according to Claim 9, wherein the above-mentioned fluid is physiological saline.

11. Method according to Claim 9 or 10, wherein the aerosol formed is irradiated with laser light having a wavelength of between 550 nm and 850 nm.

## Patentansprüche

1. Vorrichtung (1) zum Produzieren eines Aerosols zur Inhalation durch eine Person, umfassend ein Fluidreservoir (2) für ein Fluid, ein Vernebelungselement (3), das bereitgestellt wird, um das Fluid aus dem Fluidreservoir (2) in ein Aerosol umzuwandeln, und ein Ausgaberohr (4) mit einer Austragungsöffnung (5), entlang der das produzierte Aerosol ausgebbar ist, so dass es von einer Person inhaliert werden kann, wobei die Vorrichtung (1) des Weiteren einen Laser (6) umfasst, der bereitgestellt wird, um einen Lichtstrahl zu generieren, der geeignet ist, um das Aerosol während seiner Bahn durch das Ausgaberohr (4) hindurch zu bestrahlen, wobei das Ausgaberohr (4) eine Länge zwischen 100 mm und 200 mm hat, und wobei das Ausgaberohr (4) ein zulaufendes Teil (8) umfasst, wobei die Austragungsöffnung (5) in dem zulaufenden Teil bereitgestellt wird, um eine turbulente Strömung des Aerosols im Inneren des Rohrs auf Höhe des Übergangs zu dem zulaufenden Teil des Rohrs zu erzeugen, wodurch das Aerosol für einen längeren Zeitraum in Position gehalten und somit für einen längeren Zeitraum bestrahlt werden kann.

2. Vorrichtung (1) nach Anspruch 1, wobei der Laser (6) bereitgestellt wird, um einen Lichtstrahl mit einer Wellenlänge zwischen 500 nm und 1000 nm zu generieren.

3. Vorrichtung (1) nach Anspruch 1 oder 2, wobei der Laser (6) bereitgestellt wird, um einen Lichtstrahl mit einer Wellenlänge zwischen 550 nm und 850 nm zu generieren.

4. Vorrichtung (1) nach Anspruch 1, wobei der Laser (6) bereitgestellt wird, um einen Lichtstrahl mit einer Wellenlänge zwischen 400 nm und 500 nm zu generieren.

5. Vorrichtung (1) nach einem der vorhergehenden Ansprüche, wobei der Laser (6) ein Diodenlaser ist.

6. Vorrichtung (1) nach einem der vorhergehenden Ansprüche, wobei das Ausgaberohr (4) einen Zufuhrkanal (9) für Aerosol und einen Aufnahmekanal (10) umfasst, der geeignet ist, um den Laser (6) zu enthalten.

7. Vorrichtung (1) nach einem der vorhergehenden Ansprüche, wobei die Vorrichtung (1) eine Druckluftquelle (11), eine Druckluftleitung (12) zum Transportieren von Druckluft von der Druckluftquelle (10) zu dem Fluidreservoir (2) und eine Saugleitung (16) zum Aspirieren von Umgebungsluft umfasst.

8. Vorrichtung (1) nach Anspruch 7, wobei die Vorrichtung (1) ein Gehäuse (15) umfasst, das eine Filterkammer (17) zum Filtern der aspirierten Umgebungsluft umfasst.

9. Verfahren zum Erhalten eines Aerosols, das zur Behandlung von akuten und chronischen Lungenleiden geeignet ist, wobei das Verfahren die folgenden Schritte umfasst:
- Umwandeln eines Fluids in ein Aerosol; und
- Bestrahlen des gebildeten Aerosols mit Laserlicht, das eine Wellenlänge zwischen 500 nm und 1000 nm hat, wobei das gebildete Aerosol während seiner Bahn durch ein Ausgaberohr (4) mit einer Austragungsöffnung (5), entlang der das produzierte Aerosol ausgebbar ist, so dass es durch eine Person inhaliert werden kann, bestrahlt wird, wobei das Ausgaberohr (4) eine Länge zwischen 100 mm und 200 mm hat, und wobei das Ausgaberohr (4) ein zulaufendes Teil (8) umfasst, wobei die Austragungsöffnung (5) in dem zulaufenden Teil bereitgestellt wird, um eine turbulente Strömung des Aerosols im Inneren des Rohrs auf Höhe des Übergangs zu dem zulaufenden Teil des Rohrs zu erzeugen, wodurch das Aerosol für einen längeren Zeitraum in Position gehalten werden kann und somit für einen längeren Zeitraum bestrahlt wird.

10. Verfahren nach Anspruch 9, wobei das oben genannte Fluid physiologische Kochsalzlösung ist.

11. Verfahren nach Anspruch 9 oder 10, wobei das gebildete Aerosol mit Laserlicht, das eine Wellenlänge zwischen 550 nm und 850 nm hat, bestrahlt wird.

## Revendications

1. Dispositif (1) pour produire un aérosol destiné à être inhalé par une personne, comprenant un réservoir de fluide (2) pour un fluide, un élément de nébulisation (3) prévu pour convertir le fluide du réservoir de fluide (2) en un aérosol, et un tube de distribution (4) doté d'une ouverture d'évacuation (5) le long de laquelle l'aérosol produit peut être distribué afin d'être inhalé par une personne, le dispositif (1) comprenant en outre un laser (6) prévu pour générer un faisceau lumineux adapté pour irradier l'aérosol pendant sa trajectoire à travers le tube de distribution (4), le tube de distribution (4) ayant une longueur comprise entre 100 mm et 200 mm, et ledit tube de distribution (4) comprenant une partie effilée (8), l'ouverture d'évacuation (5) étant prévue dans la partie effilée, afin de créer un flux turbulent de l'aérosol à l'intérieur du tube au niveau de la transition vers la partie effilée du tube, permettant à l'aérosol d'être maintenu en place plus longtemps et donc d'être irradié pendant une plus longue période.

2. Dispositif (1) selon la revendication 1, le laser (6) étant prévu pour générer un faisceau lumineux de longueur d'onde comprise entre 500 nm et 1000 nm.

3. Dispositif (1) selon la revendication 1 ou 2, le laser (6) étant prévu pour générer un faisceau lumineux d'une longueur d'onde comprise entre 550 nm et 850 nm.

4. Dispositif (1) selon la revendication 1, le laser (6) étant prévu pour générer un faisceau lumineux de longueur d'onde comprise entre 400 nm et 500 nm.

5. Dispositif (1) selon l'une des revendications précédentes, le laser (6) étant un laser à diode.

6. Dispositif (1) selon l'une des revendications précédentes, le tube de distribution (4) comprenant un canal d'alimentation (9) en aérosol et un canal d'absorption (10) apte à recevoir le laser (6).

7. Dispositif (1) selon l'une des revendications précédentes, le dispositif (1) comprenant une source d'air comprimé (11), une conduite d'air comprimé (12) pour transporter l'air comprimé de la source d'air comprimé (10) au réservoir de fluide (2), et une conduite d'aspiration (16) pour aspirer l'air ambiant.

8. Dispositif (1) selon la revendication 7, le dispositif (1) comprenant un boîtier (15) contenant une chambre de filtrage (17) pour filtrer l'air ambiant aspiré.

9. Procédé d'obtention d'un aérosol adapté au traitement de troubles pulmonaires aigus et chroniques, lequel procédé comprenant les étapes suivantes :
- la transformation d'un fluide en aérosol ; et
- l'irradiation de l'aérosol formé avec une lumière laser d'une longueur d'onde comprise entre 500 nm et 1000 nm, l'aérosol formé étant irradié pendant sa trajectoire à travers un tube de distribution (4) doté d'une ouverture d'évacuation (5) le long de laquelle l'aérosol produit peut être distribué afin d'être inhalé par une personne, le tube de distribution (4) ayant une longueur comprise entre 100 mm et 200 mm, et ledit tube de distribution (4) comprenant une partie effilée (8), l'ouverture d'évacuation (5) étant prévue dans la partie effilée, afin de créer un flux turbulent de l'aérosol à l'intérieur du tube au niveau de la transition vers la partie effilée du tube, permettant à l'aérosol d'être maintenu en place pendant une plus longue période et donc d'être irradié pendant une plus longue période.

10. Procédé selon la revendication 9, le fluide précité étant du sérum physiologique.

11. Procédé selon la revendication 9 ou 10, l'aérosol formé étant irradié par une lumière laser dont la longueur d'onde est comprise entre 550 nm et 850 nm.
